Europäisches Patentamt

European Patent Office

Office européen des brevets

(19)

(11) Veröffentlichungsnummer: **0 066 699**
**B1**

(12) # EUROPÄISCHE PATENTSCHRIFT

(45) Veröffentlichungstag der Patentschrift:
**24.07.85**

(51) Int. Cl.⁴: **G 01 L 7/02, A 61 M 1/00**

(21) Anmeldenummer: **82103354.5**

(22) Anmeldetag: **21.04.82**

(54) **Differenzdruckanzeiger, insbesondere für medizinische Geräte.**

(30) Priorität: **10.06.81 DE 3122924**

(43) Veröffentlichungstag der Anmeldung:
**15.12.82 Patentblatt 82/50**

(45) Bekanntmachung des Hinweises auf die Patenterteilung:
**24.07.85 Patentblatt 85/30**

(84) Benannte Vertragsstaaten:
**AT BE CH DE FR GB IT LI LU NL SE**

(56) Entgegenhaltungen:
**WO - A - 81/01237**
**FR - A - 792 152**
**US - A - 3 830 238**
**US - A - 3 895 533**

(73) Patentinhaber: **INTERMEDICAT GMBH,**
**Gerliswilstrasse 43, CH-6020 Emmenbrücke (CH)**

(72) Erfinder: **Koch, Heinrich, Dr. Ing., Vockenbergsweg 5,**
**D-3509 Spangenberg (DE)**
Erfinder: **Brethauer, Ulrich, Rhönstrasse 2, D-3501 Körle**
**(DE)**

(74) Vertreter: **von Kreisler, Alek, Dipl.-Chem. et al,**
**Deichmannhaus am Hauptbahnhof, D-5000 Köln 1 (DE)**

ACTORUM AG

## Beschreibung

Die Erfindung betrifft einen Differenzdruckanzeiger, insbesondere für medizinische Geräte, z.B. für Absaugvorrichtungen für die Wunddrainage, mit einem mindestens teilweise durchsichtigen langgestreckten Gehäuse, das einen darin enthaltenen Ballon abstützt, welcher zwei gasförmige Medien voneinander trennt und eine der Druckdifferenz der Medien entsprechende Form bzw. Stellung einnimmt, und mit einem in dem Gehäuse angeordneten Anschlussstutzen, auf dem der Ballon mit seiner Öffnung befestigt ist, und der einen aus dem Gehäuse herausführenden Kanal aufweist.

Zum Absaugen von Wundsekreten und zur Adaption der Wundränder über einen in die Wunde eingelegten Drainageschlauch bei grösseren Operationswunden werden Unterdruckbehälter benutzt. Für die Kontrolle des Unterdrucks bzw. Saugdrucks sind die Behälter mit einem Differenzdruckanzeiger versehen. Dieser Differenzdruckanzeiger kann beispielsweise aus einem Faltenbalg bestehen, der sich unter der Wirkung der Druckdifferenz zwischen dem in dem Behälter herrschenden Unterdruck und dem äusseren Luftdruck verformt. Der Differenzdruckanzeiger kann an dem Behälter so angebracht sein, dass der Faltenbalg sich unter der Wirkung des Teilvakuums in dem Behälter zusammenzieht, oder er kann in den Innenraum des Behälters hineinragen, so dass er infolge des Unterdrucks gedehnt wird. Bei anderen Systemen wird eine flexible Verschlusskappe mit einem oder mehreren Anzeigehörnern eingesetzt.

Die bekannten Differenzdruckanzeiger für medizinische Geräte haben zwar den Vorteil, dass das Innere des Behälters gegenüber dem äusseren Luftdruck abgedichtet wird, sie haben jedoch gleichzeitig den Nachteil, dass nur eine qualitative Druckanzeige erfolgt und keine zuverlässige Anzeige der Grösse des Differenzdruckes.

Ein bekannter Differenzdruckanzeiger der eingangs genannten Art ist in WO-A-81/01237 beschrieben. Dieser Differenzdruckanzeiger enthält im Innern eines zweiteiligen Gehäuses einen Ballon, an dessen Spitze eine Zugfeder angreift, die den Ballon in den undurchsichtigen unteren Gehäuseteil hineinzieht. Wenn in dem unteren Gehäuseteil ein Überdruck auftritt, wird die Zugfeder gespannt und der Ballon wird unter Umfaltung in den oberen durchsichtigen Gehäuseteil hineingetrieben. Ein Mass für den Druck bzw. die Druckdifferenz zwischen unterem und oberen Gehäuseteil erhält man durch die Höhe, in der sich die ringförmige Umbiegung des Ballons im durchsichtigen Gehäuseteil befindet. Die für die Rückstellung des Ballons erforderliche elastische Kraft wird durch die Zugfeder aufgebracht, die den Differenzdruckanzeiger aufwendig und kompliziert macht. Andererseits ist die Zugfeder erforderlich, um zu erreichen, dass der Ballon sich in definierter Weise an die Wand des durchsichtigen Gehäuseteils anlegt.

Der Erfindung liegt die Aufgabe zugrunde, den Differenzdruckanzeiger der eingangs genannten Art zu vereinfachen.

Zur Lösung dieser Aufgabe ist erfindungsgemäss vorgesehen, dass der Anschlussstutzen an dem einen Ende des Gehäuses angeordnet ist und von diesem Ende aus frei in das Innere des Gehäuses hinein absteht, dass das Gehäuse an dem dem Anschlussstutzen abgewandten Ende einen nach aussen führenden Rohrstutzen aufweist, der an einen Unterdruckbehälter anschliessbar ist, und dass der Ballon in der Nähe des Anschlussstutzens einen geschwächten oder vorgedehnten ringförmigen Wandabschnitt aufweist.

Der erfindungsgemässe Differenzdruckanzeiger nutzt die Eigenschaften von Ballons oder einseitig geschlossenen Schläuchen aus, sich bei Druckbeaufschlagung zunächst nur an einer Stelle, die vorgedehnt oder in anderer Weise geschwächt ist, bis zu einem maximalen Durchmesser aufzublähen, sonst aber fast unverändert zu bleiben. Bei Drucksteigerung wächst dann diese aufgeblähte Stelle, während die übrigen Bereiche des Ballons nahezu ungedehnt bleiben. Die Länge des gedehnten Wandabschnitts kann beobachtet werden. Diese Länge bildet ein Mass für die Druckdifferenz. Der Durchmesser des abstützenden Gehäuses ist grösser als der Durchmesser des ungespannten Ballons. Beim Aufblähen legt sich der aufgeblähte Bereich des Ballons gegen die Seitenwand des Gehäuses, während der nicht aufgeblähte Bereich im Abstand von der Seitenwand verbleibt. Diejenige Länge des Gehäuses, auf der Ballon an der Gehäusewand anliegt, stellt ein Mass für den Differenzdruck dar.

Zur Erzielung der Vordehnung des Ballons in der Nähe der Ballonöffnung weist das Gehäuse eine Druckeinlassöffnung auf, an die sich im Gehäuseinnern ein Stutzen anschliesst, auf den das Endes des Ballons aufgezogen ist. Dieser Stutzen kann über seine gesamte Länge einen so grossen Durchmesser aufweisen, dass der auf ihn aufgezogene Abschnitt des Ballons gedehnt ist, er kann aber auch an seinem Ende einen erweiterten Ringkragen aufweisen. Bei Verwendung eines erweiterten Ringkragens erfolgt in dem Fall, dass keine Druckdifferenz vorhanden ist, die Vordehnung im Bereich des Ringkragens.

Gemäss einer bevorzugten Ausführungsform der Erfindung ist die Öffnung des Ballons unter Vordehnung eines Wandabschnitts eingespannt. Beim Aufblähen des Ballons wird zunächst der an den vorgedehnten Bereich anschliessende Abschnitt gedehnt, und dieser gedehnte Bereich wächst mit grösser werdender Druckdifferenz an. Wird die Druckdifferenz dann wieder kleiner, so verkleinert sich die Länge des gedehnten Bereichs schliesslich bis zur Einspannstelle hin.

Vorzugsweise ist auf den Anschlussstutzen über dem Ende des Ballons ein Schrumpfschlauch aufgeschrumpft. Dieser Schrumpfschlauch hält das Ende des Ballons fest und druckdicht an dem Stutzen.

Wenn der Differenzdruckanzeiger an einem Unterdruckbehälter für die Wunddrainage angebracht ist, muss berücksichtigt werden, dass derartige Behälter häufig längere Zeit lagern bis sie benutzt werden. Dadurch könnte infolge von Materialermüdung des Ballons die Anzeigengenauigkeit leiden. Um zu verhindern, dass der Ballon schon vor Benutzung des Unterdruckbehälters aufgrund der gegenüber dem äusseren Luftdruck herrschenden Druckdifferenz aufgebläht wird, kann in vorteilhafter Ausgestaltung der Erfindung der durch den Stutzen hindurchführende Kanal mit einem gasdichten Brechsiegel verschlossen sein. Das Siegel wird erst unmittelbar vor der Ingebrauchnahme des Behälters aufgebrochen. Vorher dichtet es den evakuierten Innenraum des Ballons gegen den äusseren Luftdruck ab. Wird das Brechsiegel entfernt, dann bläht sich der Ballon vollständig auf. Erst wenn während des Gebrauchs des Unterdruckbehälters das Vakuum verringert wird, geht der aufgeblähte Bereich des Ballons zurück.

Zur Ermöglichung einer relativ genauen Anzeige des Differenzdrucks ist gemäss einer bevorzugten Ausführungsform der Erfindung vorgesehen, dass das Gehäuse zylindrisch ist und eine längslaufende Skala aufweist.

Der Differenzdruckanzeiger kann beispielsweise auf dem Deckel eines Unterdruckbehälters stehend befestigt oder auch im Innern des Unterdruckbehälters angebracht werden.

Der Ballon bzw. Schlauch besteht vorzugsweise aus einem Latexmaterial. Um ein reibungsarmes Gleiten des Ballons an der Innenwand des Gehäuses sicherzustellen, ist die Gehäuseinnenwand vorzugsweise mit einer dünnen Silikonölschicht beschichtet.

Im folgenden wird unter Bezugnahme auf die Zeichnungen ein Ausführungsbeispiel der Erfindung näher erläutert.

Es zeigen:

Figur 1 einen Längsschnitt durch den Differenzdruckanzeiger im drucklosem Zustand,

Figur 2 einen schematischen Längsschnitt bei einem relativ geringen Differenzdruck, und

Figur 3 einen schematischen Längsschnitt bei grösserem Differenzdruck.

Der Differenzdruckanzeiger weist ein langgestrecktes zylindrisches Gehäuse 10 aus durchsichtigem Kunststoff auf. Von der Bodenwand 11 des Gehäuses 10 steht ein koaxial zu dem Gehäuse verlaufender Stutzen 12 ab, dessen freies Ende einen nach aussen erweiterten Ringkragen 13 aufweist. Über den Ringkragen 13 ist das Ende des Ballons 14 auf den Stutzen 12 aufgezogen. Der Aussendurchmesser des Stutzens 12 ist grösser als der Durchmesser des Ballons 14 im ungedehnten Zustand, so dass das auf den Stutzen 12 und den Ringkragen 13 aufgezogene Ende des Ballons radial gedehnt wird. Hinter dem Ringkragen 13 ist ein Schrumpfschlauch 20 über dem auf dem Stutzen 12 befindlichen Ende des Ballons befestigt. Der Schrumpfschlauch 20 hält die Ballonöffnung somit auf dem Stutzen 12 fest.

Die Länge des Gehäuses 10 verhält sich zu der Länge des Ballons 14 im ungedehnten Zustand etwa wie 3:1. Ebenso beträgt das Verhältnis des Gehäusedurchmessers zum Durchmesser des Ballons 14 im ungedehnten Zustand etwa 3:1.

Der durch den Stutzen 12 hindurchgehende Kanal 15 ist nach aussen durch ein Rohrstück 16 verlängert, dessen Öffnung mit einem Brechsiegel gasdicht abgeschlossen werden kann.

An der der Bodenwand 11 abgewandten Deckelwand 17 befindet sich ein Rohrstutzen 18, mit einem in das Innere des Gehäuses 10 hineinführenden Kanal. Im Falle des Anschlusses des Differenzdruckanzeigers an einen Unterdruckbehälter wird der Rohrstutzen 18 mit dem Innern des Unterdruckbehälters verbunden, während der Rohrstutzen 16 mit der Aussenluft verbunden ist. Dabei strafft sich der Ballon 14 zwar in Längsrichtung, bläht sich aber nur ausgehend von der schon vorgedehnten Stelle in der Nähe der Ballonöffnung auf, bis er die Innenwand des Gehäuses 10 berührt.

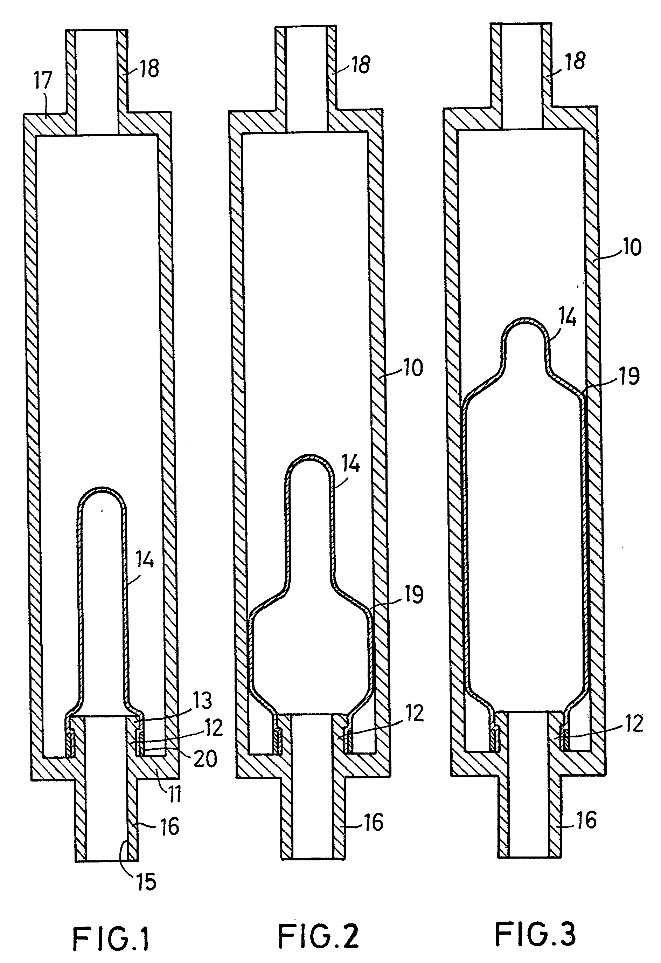

Dabei ist die Länge des aufgeblähten Teiles ein Mass für die Druckdifferenz zwischen dem Innenraum des Ballons 14 und dem Innenraum des Gehäuses 10. Die Länge des aufgeblähten Teiles des Ballons 14 nimmt mit grösser werdender Druckdifferenz zu. Dies ist in den Figuren 2 und 3 dargestellt. An der durchsichtigen Aussenwand des Gehäuses 10 befindet sich eine (nicht dargestellte) Skala, an der die Höhe des jeweiligen Anlagerandes 19 des Ballons 14 an dem Gehäuse abgelesen werden kann. Die Längenmarkierungen an der Skala können so gewählt werden, dass der Druck direkt abgelesen werden kann. Der Anzeigebereich des Differenzdruckanzeigers hängt von den gewählten Längen und Durchmessern, der Wandstärke und den elastischen Eigenschaften des Ballons ab.

**Patentansprüche**

1. Differenzdruckanzeiger, insbesondere für medizinische Geräte, z.B. für Absaugvorrichtungen für die Wunddrainage, mit einem mindestens teilweise durchsichtigen langgestreckten Gehäuse (10), das einen darin enthaltenen Ballon (14) abstützt, welcher zwei gasförmige Medien voneinander trennt und eine der Druckdifferenz der Medien entsprechende Form bzw. Stellung einnimmt, und mit einem in dem Gehäuse (10) angeordneten Anschlussstutzen (12), auf dem der Ballon (14) mit seiner Öffnung befestigt ist, und der einen aus dem Gehäuse (10) herausführenden Kanal (15) aufweist, dadurch gekennzeichnet, dass der Anschlussstutzen (12) an dem einen Ende des Gehäuses (10) angeordnet ist und von diesem Ende aus frei in das Innere des Gehäuses hinein absteht, dass das Gehäuse (10) an dem dem Anschlussstutzen (12) abgewandten Ende einen nach aussen führenden Rohrstutzen (18) aufweist, der an einen Unterdruckbehälter anschliessbar ist, und dass der Ballon (14) in der Nähe des Anschlussstutzens (12) einen geschwächten oder vorgedehnten ringförmigen Wandabschnitt aufweist.

2. Differenzdruckanzeiger nach Anspruch 1, dadurch gekennzeichnet, dass die Öffnung des Ballons (14) unter Vordehnung eines Wandabschnitts eingespannt ist.

3. Differenzdruckanzeiger nach Anspruch 2, dadurch gekennzeichnet, dass der Stutzen (12) an seinem Ende einen erweiterten Ringkragen (13) aufweist.

4. Differenzdruckanzeiger nach einem der Ansprüche 1 bis 3, dadurch gekennzeichnet, dass auf den Anschlussstutzen (12) über dem Ende des Ballons (14) ein Schrumpfschlauch (20) aufgeschrumpft ist.

5. Differenzdruckanzeiger nach einem der vorhergehenden Ansprüche, dadurch gekennzeichnet, dass der durch den Anschlussstutzen (12) hindurchgehende Kanal (15) mit einem gasdichten Brechsiegel verschlossen ist.

6. Differenzdruckanzeiger nach einem der vorhergehenden Ansprüche, dadurch gekennzeichnet, dass das Gehäuse (10) zylindrisch ist und eine längslaufende Skala aufweist.

**Revendications**

1. Indicateur de pression différentielle, en particulier pour des appareils médicaux tels que des dispositifs d'aspiration pour le drainage des plaies, comportant un boîtier au moins en partie transparent (10) de forme allongée, qui soutient un ballon (14) contenu dans ce boîtier et séparant deux milieux gazeux; ce ballon pouvant ainsi se déformer, et prendre une forme et/ou une position correspondant à la différence de pression entre les deux fluides; l'indicateur comportant en outre un embout de raccordement (12) disposé dans le boîtier (10), sur lequel est fixé le ballon (14) par l'ouverture de celui-ci; cet embout (12) étant percé d'un canal (15) qui débouche à l'extérieur du boîtier (10); caractérisé en ce que l'embout de raccordement (12) est disposé à l'une des extrémités du boîtier (10), et se trouve librement en saillie, à partir de cette extrémité, vers l'intérieur du boîtier; en ce que le boîtier (10), à l'endroit de son autre extrémité opposée à l'embout de raccordement (12), porte un appendice tubulaire (18) qui débouche à l'extérieur, et qui peut être raccordé à un récipient en dépression; et en ce que le ballon (14) présente une zone annulaire amincie ou ayant subi une extension préalable, au voisinage de l'embout de raccordement (12).

2. Indicateur selon la revendication 1, caractérisé en ce que l'ouverture du ballon (14) est fixée en place par serrage, sous l'effet d'une extension préalable d'une partie de la paroi du ballon.

3. Indicateur selon la revendication 2, caractérisé en ce que l'embout de raccordement (12) porte à son extrémité une collerette annulaire élargie (13).

4. Indicateur selon l'une des revendications 1 à 3, caractérisé en ce que l'extrémité du ballon (14) est fixée par serrage sur l'embout de raccordement (12), au moyen d'un tube rétractable (20) disposé sur l'extrémité du ballon (14).

5. Indicateur conforme à l'une des revendications 1 à 4, caractérisé en ce que le canal (15) qui passe à travers l'embout de raccordement (12) est obturé par une capsule étanche aux gaz et que l'on peut briser.

6. Indicateur conforme à l'une des revendications 1 à 5, caractérisé en ce que le boîtier (10) est cylindrique, et porte une échelle de lecture longitudinale.

**Claims**

1. Differential pressure indicator, especially for medical apparatuses such as sucking apparatuses for the drainage of wounds, comprising an at least partly transparent, elongated housing (10) supporting a balloon (14) contained in it which separates two gaseous fluids and takes a shape or position corresponding to the pressure difference of the fluids, and a connecting piece (12) provided in the housing (10) and on which the balloon is fixed with its aperture, the connecting piece having a channel (15) extending out of the housing (10), characterized in that the connecting piece (12) is disposed at the one end of the housing (10) and projects freely from said end into the inside of the housing, that at its end averted from the connecting piece (12), the housing (10) comprises a tubular piece (18) extending to the outside and being connectible to a vacuum container, and that the balloon (14) contains near the connecting piece (12) a weak or prestretched annular wall section.

2. Differential pressure indicator according to claim 1, characterized in that the aperture of the balloon (14) is fixed by prestretching a wall section.

3. Differential pressure indicator according to claim 2, characterized in that the end of the connecting piece (12) contains a flared annular collar (13).

4. Differential pressure indicator according to one of claims 1 to 3, characterized in that a shrunk hose (20) is applied by shrinking on the connecting piece (12) above the end of the balloon (14).

5. Differential pressure indicator according to one of the preceding claims characterized in that the channel (15) extending through the connecting piece (12) is closed by a gas-tight breaking seal.

6. Differential pressure indicator according to one of the preceding claims characterized in that the housing (10) is cylindrical and graduated longitudinally.

FIG.1    FIG.2    FIG.3